# EUROPEAN PATENT SPECIFICATION

(11) **EP 1 493 424 B1**
(45) Date of publication and mention of the grant of the patent: **21.01.2009**
(21) Application number: 03745959.1
(22) Date of filing: 08.04.2003
(51) Int. Cl.: A61K 8/41, A61K 8/42, A61K 8/44, A61Q 5/02, A61Q 5/12

(54) **COSMETIC HAIR PREPARATION**
KOSMETISCHES HAARPRÄPARAT
PREPARATION COSMETIQUE POUR CHEVEUX

(30) Priority: 08.04.2002 JP 2002105848
(43) Date of publication of application: 05.01.2005
(73) Proprietor: KAO CORPORATION, Tokyo 103-8210 (JP)
(72) Inventor: DOI, Yasuhiro, Wakayama-Shi, Wakayama 640-8580 (JP); HASEBE, Keiko, Wakayama-Shi, Wakayama 640-8580 (JP)
(74) Representative: HOFFMANN EITLE
(86) International application number: PCT/JP2003/004446
(87) International publication number: WO 2003/084492

(56) References cited:
- EP-A1- 1 283 030
- EP-A2- 1 219 290
- WO-A-99/60082
- GB-A- 1 226 801
- DATABASE WPI Week 200143 Derwent Publications Ltd., London, GB; AN 2001-401121 XP002444034 & JP 2001 081015 A (FANKERU KK) 27 March 2001 (2001-03-27)

## Description

### TECHNICAL FIELD

The present invention relates to hair cosmetic compositions capable of giving good flexibility and smoothness to the hair during the period from wetting to drying and even after drying, particularly giving excellent flexibility and smoothness to the hair after drying.

### BACKGROUND ART

Hair cosmetic compositions are required to be able to give flexibility and smoothness to the hair during the period from wetting to drying and even after drying. In order to satisfy this requirement, a quaternary ammonium salt such as stearyltrimethylammonium chloride, behenyltrimethylammonium chloride or distearyldimethylammonium chloride is incorporated in the compositions as a cationic surfactant. This attempt does not always lead to success in the preparation of satisfactory hair cosmetic compositions.

Processes using a variety of additives such as silicone to improve the smoothness after drying are known. These additives however adversely affect the stability of the system and therefore need adjustment of their amount or cumbersome blending work.

It is disclosed in Japanese Patent Laid-Open No. 2001-81015 that use of a quaternary ammonium salt and a tertiary amine salt in combination is effective for giving moist feel to the hair. However, hair cosmetic compositions capable of giving flexibility and smoothness to the hair during the period from wetting to drying and even after drying have not yet been obtained.

EP 1 283 030 A1 (prior art according to Art. 54(3) EPC) describes a conditioner composition comprising a tertiary amine and optionally an inorganic or organic acid.

EP 1 219 290 A2 (prior art according to Art. 54(3) EPC) reveals a hair cosmetic composition comprising an ether type cationic surfactant.

An object of the present invention is to provide hair cosmetic compositions capable of giving good flexibility and smoothness to the hair during the period from wetting to drying and even after drying.

### DISCLOSURE OF THE INVENTION

The present inventors have found that a hair cosmetic composition capable of satisfying the above-described demand can be obtained by using a specific ether type cationic surfactant and a tertiary amine and/or salt thereof.

In the present invention, there is thus provided a hair cosmetic composition containing the following components (A) and (B):

(A) an ether type cationic surfactant represented by the following formula (1): 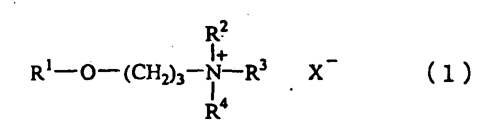 (wherein, R¹ represents a linear or branched alkyl or alkenyl group having 6 to 24 carbon atoms,

R² and R⁴ each represents an alkyl group having 1 to 6 carbon atoms or -(A¹O)ₙH (in which A¹ represents an alkylene group having 2 to 4 carbon atoms, n stands for 1 to 6, and n pieces of A¹ may be the same or different and arranged in any order),

R³ represents an alkyl group having 1 to 6 carbon atoms, a benzyl group or -(A²O)ₘH (in which A² represents an alkylene group having 2 to 4 carbon atoms, m stands for 1 to 6, and m pieces of A² may be the same or different and arranged in any order), and X⁻ represents an anion), and

(B) at least one compound selected from tertiary amines each represented by the following formula (2): 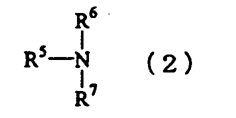 (wherein, R⁵ represents a linear or branched alkyl or alkenyl group having 6 to 24 carbon atoms, or R⁸-Q¹-R⁹ (in which, R⁸ represents a linear or branched alkyl or alkenyl group having 6 to 24 carbon atoms, Q¹ represents a functional group selected from -OCO-, -COO-, -CONH-, -NHCO-, -NH-CO-NH-, and -(A³O)ₛ- (in which A³ represents an alkylene group having 2 to 4 carbon atoms, s stands for 1 to 24, s pieces of A³ may be the same or different and arranged in any order) and R⁹ represents an alkylene group having 1 to 6 carbon atoms),

R⁶ represents a linear or branched alkyl or alkenyl group having 1 to 24 carbon atoms, -(A⁵O)ₖH (in which A⁵ represents an alkylene group having 2 to 4 carbon atoms, k stands for 1 to 6, k pieces of A⁵ may be the same or different and arranged in any order) or R²⁸-Q²-R²⁹ (in which R²⁸ represents a linear or branched alkyl or alkenyl group having 6 to 24 carbon atoms, Q² represents a functional group selected from -OCO-, -COO-, -CONH-, -NHCO-, -NH-CO-NH-, and -(A⁴O)ₜ- (in which A⁴ represents an alkylene group having 2 to 4 carbon atoms, t stands for 1 to 24, t pieces of A⁴ may be the same or different and arranged in any order) and R²⁹ represents an alkylene group having 1 to 6 carbon atoms), and

R⁷ represents a linear or branched alkyl group having 1 to 6 carbon atoms or - (A⁶O)ᵤH (in which A⁶ represents an alkylene group having 2 to 4 carbon atoms, u stands for 1 to 6, u pieces of A⁶ may be the same or different and arranged in any order), and/or salts of the tertiary amines.

### BEST MODE FOR CARRYING OUT THE INVENTION

In the formula (1) of Component (A), R¹, R², R³ and R⁴ representing the below-described groups are preferred from the viewpoints of giving flexibility and smoothness to the hair during the period from wetting to drying and even after drying.

In the formula (1), as R¹, linear or branched alkyl or alkenyl groups having from 12 to 22, more preferably from 16 to 18 carbon atoms are preferred, of which the linear alkyl groups are more preferred.

As R² and R⁴, alkyl groups having 1 to 6 carbon atoms and -(CH₂CH₂O)ₙH are preferred. In the group - (CH₂CH₂O)ₙH, n preferably stands for 1 to 3, more preferably 1. Of these, methyl and ethyl groups are more preferred, with methyl being more preferred.

As R³, methyl, ethyl and benzyl groups are preferred, of which methyl and ethyl groups are more preferred, with methyl being more preferred.

Examples of X⁻ include halogen ions and organic anions such as acetate, citrate, lactate, glycolate, phosphate, nitrate, sulfonate, sulfate and alkyl sulfates, for example, ethyl sulfate. Of these, a chlorine ion is more preferred.

In the formula (2) representing Component (B), (i) mono(long chain) tertiary amines and/or salts thereof or (ii) di(long chain) tertiary amines and/or salts thereof are preferred, because they can give flexibility to the hair upon wetting. Of these, (i) mono(long chain) tertiary amines and/or salts thereof are more preferred. (i) Mono(long chain) tertiary amines and/or salts thereof

In the formula (2), R⁵ preferably represents a linear or branched alkyl group having 12 to 22 carbon atoms, or R⁸-Q¹-R⁹ (in which, R⁸ represents a linear or branched alkyl or alkenyl group having 8 to 22 carbon atoms, Q¹ represents a functional group selected from -OCO-, -COO-, - CONH-, -NHCO-, -NH-CO-NH-, and -(A³O)ₛ- (in which A³ represents an alkylene group having 2 to 3 carbon atoms, s stands for 1 to 24, s pieces of A³ may be the same or different and arranged in any order) and R⁹ represents an alkylene group having 1 to 3 carbon atoms), more preferably a linear or branched alkyl group having from 16 to 22 carbon atoms or R⁸-Q¹-R⁹ (in which, R⁸ represents a linear or branched alkyl or alkenyl group having from 12 to 22 carbon atoms, Q¹ represents a functional group selected from -OCO-, -COO-, -CONH- and -NHCO- and R⁹ represents an alkylene group having 1 to 3 carbon atoms).

As R⁶ and R⁷, linear or branched alkyl groups having 1 to 6 carbon atoms and -CH₂CH₂O)ᵣH are preferred. In the group -(CH₂CH₂O)ᵣH, r preferably stands for 1 to 3, preferably 1. Of these, methyl and ethyl groups are still more preferred, with methyl being more preferred. (ii) Di(long chain) tertiary amine and/or salt thereof

As R⁶, preferred are linear or branched alkyl groups having 12 to 22 carbon atoms and R²⁸-Q²-R²⁹ (in which, R²⁸ represents a linear or branched alkyl or alkenyl group having 8 to 22 carbon atoms, Q² represents -OCO-, -COO-, - CONH-, -NHCO-, -NH-CO-NH-, or -(A⁴O)ₜ- (in which A⁴ represents an alkylene group having 2 to 3 carbon atoms, t stands for 1 to 24, t pieces of A⁴ may be the same or different and arranged in any order) and R²⁹ represents an alkylene group having 1 to 3 carbon atoms), with linear or branched alkyl groups having 16 to 22 carbon atoms and R²⁸-Q²-R²⁹ (in which, R²⁸ represents a linear or branched alkyl or alkenyl group having 12 to 18 carbon atoms, Q² represents a functional group selected from -OCO-, -COO-, - CONH- or -NHCO-, and R²⁹ represents an alkylene group having 1 to 3 carbon atoms) being more preferred. In this case, as R⁵ and R⁷, those exemplified above are preferred.

In the invention, the above-described hair cosmetic composition may further contain at least one acid selected from inorganic acids and organic acids to reduce an amine odor or heighten conditioning effects such as flexibility and smoothness.

Examples of the inorganic acid include hydrochloric acid, sulfuric acid and phosphoric acid, while those of the organic acid include monocarboxylic acids such as acetic acid and propionic acid, dicarboxylic acids such as malonic acid, succinic acid, glutaric acid, adipic acid, maleic acid, fumaric acid and phthalic acid, hydroxycarboxylic acids such as glycolic acid, lactic acid, hydroxyacrylic acid, glyceric acid, malic acid, tartaric acid and citric acid, polycarboxylic acids such as polyglutamic acid, and acidic amino acids such as glutamic acid and aspartic acid. Of these, inorganic acids, dicarboxylic acids, hydroxycarboxylic acids and acidic amino acids are preferred. As the inorganic acid, hydrochloric acid is preferred. As the dicarboxylic acid, maleic acid and succinic acid are preferred. As the hydroxycarboxylic acid, glycolic acid, citric acid, lactic acid and malic acid are preferred. As the acidic amino acid, glutamic acid is preferred.

The above-described inorganic acid and/or organic acid are(is) preferably added in an amount of from 0.1 to 10 moles, more preferably 0.3 to 3 moles per mole of the tertiary amine or salt thereof.

When the hair cosmetic composition contains at least one acid selected from the inorganic acids and/or organic acids, the tertiary amine may form a salt therewith or may exist without forming a salt.

Examples of the salt of the tertiary amine include salts with the above-described inorganic acids or organic acids. Hydrochlorides, lactates, malates and glutamates are preferred.

The total content of Components (A) and (B) in the hair cosmetic composition of the invention is preferably from 0.1 to 20 wt.% in order to give sufficient flexibility and smoothness to the hair and maintain stability of the product without generating precipitation, caking or layer separation upon storage. When the hair cosmetic composition is a rinse-off type such as hair rinse and hair conditioner, that from 0.2 to 10 wt.% is preferred, while when it is a leave-on type such as leave-on hair treatment or hair liquid, that from 0.2 to 5 wt.% is preferred.

A weight ratio of Component (A) to (B), that is, Component (A)/(B) weight ratio is preferably from 20/1 to 1/20, more preferably from 5/1 to 1/5, even more preferably from 2/1 to 1/2 from the viewpoint of good exhibition of flexibility and smoothness.

The content of Component (A) in the hair cosmetic composition is usually from 0.1 to 20 wt.%, preferably from 0.2 to 5 wt.%. The content of Component (B) in the hair cosmetic composition is usually from 0.1 to 20 wt.%, preferably from 0.2 to 5 wt.%.

The hair cosmetic composition of the invention may contain a surfactant other than Component (A) or (B). As the other surfactant, at least one of cationic surfactants other than Component (A) or (B), anionic surfactants, nonionic surfactants and amphoteric surfactants can be used. Of these, cationic surfactants are preferred.

Examples of the preferred cationic surfactants other than Component (A) or (B) include quaternary ammonium salts as described in Japanese Patent Laid-Open No. 2000-178146 and represented by the following formula (3): 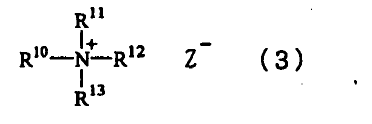 (wherein, at least one of R¹⁰, R¹¹, R¹² and R¹³ represents a linear or branched alkyl or alkenyl group which may be substituted by a linear or branched alkoxy group, alkenyloxy group, alkanoylamino group, alkenoylamino group, alkanoyloxy group or alkenoyloxy group and has 12 to 28, preferably 16 to 28 carbon atoms in total, and the remaining one(s) represents a benzyl, alkyl having from 1 to 5 carbon atoms, hydroxylalkyl or polyoxyethylene group having a total molar addition number not more than 10, and Z⁻ represents a halogen ion or an organic anion, for example, selected from acetate, citrate, lactate, glycolate, phosphate, nitrate, sulfonate, sulfate and alkylsulfate groups).

Among the above-described compound (3), preferred are those in which at least one of R¹⁰, R¹¹, R¹² and R¹³ is an alkyl group which may be substituted with an alkoxy group and has 12 to 22 carbon atoms in total and the remaining group(s) is(are) a methyl, ethyl or benzyl group. More preferred examples include mono(long-chain alkyl)trimethylammonium chloride and di(long-chain alkyl)dimethylammonium chloride.

As the above-described anionic surfactant, sulfates, sulfonates, caboxylates, phosphates and amino acids are preferred. Specific examples include alkyl sulfates, polyoxyalkylene alkyl ether sulfates, polyoxyalkylene alkenyl ether sulfates, alkyl sulfosuccinates, polyoxyalkylene alkyl sulfosuccinates, polyoxyalkylene alkyl phenyl ether sulfates, alkanesulfonates, acyl isethionates, acyl methyl taurates, higher fatty acid salts, polyoxyalkylene alkyl ether acetates, alkyl phosphates, polyoxyalkylene alkyl ether phosphates, acyl glutamates, alanine derivatives, glycine derivatives and arginine derivatives.

Among these, polyoxyethylene alkyl ether sulfates, polyoxyethylene alkenyl ether sulfates, alkyl sulfates, acyl isethionates, acyl methyl taurates, higher fatty acid salts, polyoxyalkylene alkyl ether acetates, alkyl phosphates, polyoxyalkylene alkyl ether phosphates, alkyl acyl glutamates and alkyl alanine derivatives are preferred, with those represented by the formula (4) or (5) being more preferred.

R¹⁴O(CH₂CH₂O)ₚSO₃M (4)

R¹⁵OSO₃M (5)

(wherein, R¹⁴ represents an alkyl or alkenyl group having 10 to 18 carbon atoms, R¹⁵ represents an alkyl group having 10 to 18 carbon atoms, M represents an alkali metal, alkaline earth metal, ammonium, alkanolamine or basic amino acid, and p stands for an average molar addition number of ethylene oxide and is a number of from 1 to 5).

Examples of the nonionic surfactant include polyoxyalkylene sorbitan fatty acid esters, polyoxyalkylene sorbitol fatty acid esters, polyoxyalkylene glycerin fatty acid esters, polyoxyalkylene fatty acid esters, polyoxyalkylene alkyl ethers, polyoxyalkylene alkyl phenyl ethers, polyoxyalkylene (hydrogenated) castor oils, sucrose fatty acid esters, polyglycerin alkyl ethers, polyglycerin fatty acid esters, fatty acid alkanolamides and alkyl glycosides. Of these, alkyl glycosides, polyoxyalkylene C₈-₂₀ fatty acid esters, polyoxyethylene sorbitan fatty acid esters, polyoxyethylene hydrogenated castor oils and fatty acid alkanolamides are preferred. As alkyl glycosides, those having a C₈₋₁₄ alkyl group and a sugar (glucose) condensation degree of from 1 to 2 are preferred. As the fatty acid alkanolamides, those having an acyl group with 8 to 18 carbon atoms, especially 10 to 16 carbon atoms are preferred. The fatty acid alkanolamide may be a monoalkanolamide or dialkanolamide, but that having a hydroxyalkyl group with from 2 to 3 carbon atoms is preferred. Specific examples of the fatty acid alkanolamide include oleic diethanolamide, palm-kernel fatty acid diethanolamide, coconut fatty acid diethanolamide, lauric diethanolamide, polyoxyethylene coconut fatty acid monoethanolamide, coconut fatty acid monoethanolamide, lauric monoisopropanolamide, lauric monoethanolamide, palm-kernel fatty acid methyl ethanolamide and coconut fatty acid methyl ethanolamide.

As the amphoteric surfactant, betaine surfactants can be used. Of these, imidazoline betaines, alkyldimethyl aminoacetic acid betaines, fatty acid amidopropylbetaines and alkylhydroxy sulfobetaines are more preferred, with alkylcarboxymethylhydroxyethyl imidazolium betaines, fatty acid amidopropylbetaines and alkylhydroxy sulfobetaines being more preferred. As the fatty acid amidopropylbetaines and alkylhydroxy sulfobetaines, those having an alkyl group with from 8 to 18, especially from 10 to 16 carbon atoms are preferred, of which lauric amidopropylbetaine, palm-kernel fatty acid amidopropylbetaines, coconut fatty acid amidopropylbetaines and lauryl hydroxy sulfobetaine are still more preferred.

The using ratio of the total amount of Components (A) and (B) to the other surfactant, that is, "total of Components (A) and (B)/the other surfactant" (weight ratio) is preferably 1/200 or greater, more preferably from 1/200 to 10/1, even more preferably from 1/100 to 4/1 from the viewpoint of flexibility and smoothness after drying.

The using ratio of the total amount of Components (A) and (B) to the other cationic surfactant, that is "total of Components (A) and (B)/the other cationic surfactant" (weight ratio) is preferably from 1/4 or greater, more preferably from 1/2 to 10/1, even more preferably from 1/1 to 4/1.

When the hair cosmetic composition of the present invention is a hair cleansing composition such as shampoo, the total content of Components (A) and (B) is preferably from 0.1 to 20 wt.%, more preferably from 0.2 to 10 wt.%, even more preferably from 0.5 to 5 wt.% from the viewpoint of improving foam quality and stability. The content of the anionic surfactant, nonionic surfactant or amphoteric surfactant is preferably from 0.1 to 50 wt.%, more preferably from 0.5 to 30 wt.%, even more preferably from 5 to 20 wt.% from the viewpoint of improving foaming property and feeling upon use.

The using ratio of the total amounts of Components (A) and (B) to the anionic surfactant, nonionic surfactant or amphoteric surfactant, that is, "total amount of Components (A) and (B)/the other anionic surfactant, nonionic surfactant or amphoteric surfactant" (weight ratio) is preferably from 1/1 to 1/200, more preferably from 1/5 to 1/100 from the viewpoint of improving foam quality and stability.

The hair cosmetic composition of the invention may further contain an oil component. Examples of the oil component include higher alcohols, ester oils, silicones, hydrocarbons and glycerides, of which higher alcohols, ester oils and/or silicones are preferred, with higher alcohols and/or silicones being more preferred.

The higher alcohols include those having a linear or branched alkyl or alkenyl group, preferably those having a linear or branched alkyl or alkenyl group having 12 to 26 carbon atoms. More preferred examples thereof include cetanol, cetyl alcohol, stearyl alcohol, isostearyl alcohol, oleyl alcohol, arachidic alcohol, behenyl alcohol, carnaubyl alcohol and ceryl alcohol. Of these, cetanol, cetyl alcohol, stearyl alcohol and behenyl alcohol are more preferred. The term "cetanol" as used herein means an alcohol composed mainly of a cetyl alcohol and containing a higher alcohol such as stearyl alcohol or oleyl alcohol.

As the ester oil, ester oils having 8 to 40 carbon atoms in total, preferably, esters composed of a fatty acid having 8 to 20 carbon atoms in total and an alcohol having 1 to 20 carbon atoms can be used. Among these, isopropyl palmitate and isopropyl myristate are more preferred.

Examples of the silicones include (a) dimethylpolysiloxane, (b) methylphenylpolysiloxane, (c) amino-modified silicones (preferably, those having an average molecular weight of about 3000 to 100000 and described under the name "Amodimethicone" in CTFA Dictionary (USA, Cosmetic Ingredient Dictionary), the 3rd edition, and aqueous emulsions such as "SM8704C" (product of Dow Corning Toray Silicone) and "DC939" (product of Dow Corning Toray Silicone), (d) fatty acid-modified polysiloxanes, (e) alcohol-modified silicones, (f) aliphatic alcohol-modified polysiloxanes, (g) polyether-modified silicones, (h) epoxy-modified silicones, (i) fluorine-modified silicones, (j) cyclic silicones, and (k) alkyl-modified silicones.

When the hair cosmetic composition of the invention is a rinse-off type such as a hair shampoo, hair rinse or a hair conditioner, the silicones (a), (c), (f), (g) and (j) are preferred, while when it is a leave-on type such as a hair cream or a leave-on-treatment, the silicones (a), (b), (c), (g) and (j) are preferred.

The content of the oil component in the hair cosmetic composition of the invention is preferably from 0.01 to 30 wt.%, more preferably from 0.1 to 30 wt.%, still more preferably from 0.2 to 20 wt.%, even more preferably from 1 to 20 wt.% in view of providing the hair with sufficient flexibility and moist feel peculiar to the oil component and also from the viewpoint of the stability of the product. Above all, addition of the silicone in an amount of from 0.01 to 20 wt.%, preferably from 0.1 to 10 wt.% is preferred, because feeling or touch peculiar to silicones can be imparted to the hair and a stable product can be obtained.

In the hair cosmetic composition of the invention, a weight ratio of the total amount of Components (A) and (B) to the amount of the oil component, that is, a weight ratio of total amounts of Components (A) and (B)/the amount of the oil component preferably ranges from 20/1 to 1/30, more preferably from 10/1 to 1/10, even more preferably from 1/1 to 1/10 from the viewpoint of stabilizing the emulsified state of the oil components. A weight ratio of the sum of the total amounts of Components (A) and (B) and the oil component other than the silicone to the amount of the silicone, that is, (total amount of Components (A) and (B) + the amount of the oil component other than silicone)/the amount of the silicone preferably ranges from 20/1 to 1/20, more preferably from 10/1 to 1/10, more preferably from 10/1 to 1/1 from the viewpoint of the stability of the product.

Although no particular limitation is imposed on the pH of the hair cosmetic composition of the invention, the pH at 25°C of the composition diluted 20-folds with water is preferably from 2 to 8. When it is used as a hair rinse or conditioner, the pH (at 25°C) is preferably from 3 to 6, while when it is used as a hair shampoo, the pH (at 25°C) is from 5 to 8. The pH may be adjusted by the addition of an acid or an alkali.

The hair cosmetic composition of the invention may further contain additives other than the above-described oil component, such as vegetable oils, animal oils, lanoline derivatives, higher fatty acid esters, higher fatty acids; glycerin, humectants, cationic polymers, polysaccharides, polypeptides, pearlescent agents, solvents, liquid-crystal forming bases, aromatic sulfonic acids, colorants, perfumes, propellants, chelating agents, pH regulators, antiseptics and anti-dandruffs as needed. Specific examples of the vegetable oils include camellia oil, Macadamia nut oil, mink oil, olive oil, safflower oil, soybean oil and jojoba oil. Those of the cationic polymer include cationic cellulose derivatives, cationic starch and cationic guar gum derivatives. Those of the antidandruff include zinc pyrithione and piroctone olamine.

The hair cosmetic composition of the present invention can be prepared in a conventional manner to a desired form such as aqueous solution, ethanol solution, emulsion, suspension, gel, liquid crystal, solid, aerosol foam or spray. The hair cleansing composition can be provided as a hair shampoo and the like. Examples of the products of the hair cosmetic composition other than the hair cleansing composition include hair rinses, hair conditioners, hair treatments, hair packs, hair creams, hair colors, conditioning mousses, hair mousses, hair sprays, leave-on-treatments, waxes, tonics, hair water and hair dyes.

### Examples

The present invention will hereinafter be described in further detail by way of Examples.

### Example 1

Hair conditioners (Invention Products 1 to 11 and Comparative Products 1 to 3) having the compositions as shown in Tables 3 to 4 were prepared in a conventional manner by using ether type cationic surfactants 1 to 4 as shown in Table 1 and tertiary amine salts as shown in Table 2. Flexibility of these hair conditioners was organoleptically evaluated in accordance with the below-described method. The numerals in Tables 3 to 4 are by wt.%.

**Table 1**

| Ether type cationic surfactant linear) | Formula (1) | | | | |
|---|---|---|---|---|---|
| | R¹ (each, | R² | R³ | R⁴ | X⁻ |
| 1 | C₁₆H₃₃/C₁₈H₃₇ =50/50 (wt.%) | CH₃ | CH₃ | CH₃ | Cl⁻ |
| 2 | C₁₆H₃₃ | CH₃ | CH₂CH₃ | CH₃ | CH₃CH₂OSO₃⁻ |
| 3 | C₁₈H₃₇ | CH₃ | CH₃ | CH₃ | Cl⁻ |
| 4 | C₁₈H₃₇ | CH₂CH₂OH | CH₃ | CH₂CH₂OH | Cl⁻ |

**Table 2**

| Tertiary amine salt | Structural formula |
|---|---|
| Tertiary amine salt 1 | 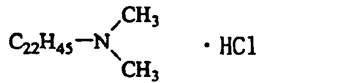 |
| Tertiary amine salt 2 | 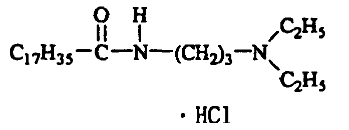 |
| Tertiary amine salt 3 | 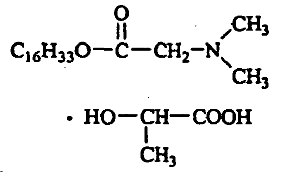 |
| Tertiary amine salt 4 | 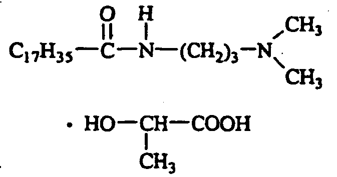 |
| Tertiary amine salt 5 | 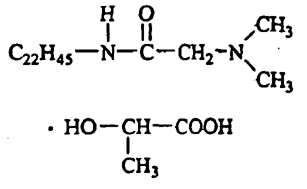 |

### <Evaluation method>

A bundle (20 cm long, 20 g) of the Japanese female hair, which had never been subjected to cold permanent waving or bleaching, was shampooed with a commercially available shampoo composed mainly of an anionic surfactant. To the resulting hair bundle, 1.0 g of a hair conditioner was applied uniformly, followed by rinsing with running water of 40°C for 30 seconds. Flexibility and smoothness of the wet hair upon rinsing, and those of the hair after sufficient drying with a dryer were evaluated by a panel of 5 experts in accordance with the following standards.
4: excellent, 3: good, 2: hard to say either way, 1: bad
An average score of five experts was calculated. The hair conditioner of 3.6 or greater was rated as A, that of 2.6 to 3.4 was rated as B, that of 1.6 to 2.4 was rated as C, and that of 1.4 or less was rated as D. The results are shown in Tables 3 and 4.

**Table 3**

| Composition of hair rinse (%) | | | Invention products | | | | | | | | | | |
|---|---|---|---|---|---|---|---|---|---|---|---|---|---|
| | | | 1 | 2 | 3 | 4 | 5 | 6 | 7 | 8 | 9 | 10 | 11 |
| Ether type cationic surfactant 1 | | | 1.0 | | | | | | 1.0 | | | | |
| Ether type cationic surfactant 2 | | | | 1.0 | | | | | | | | | |
| Ether type cationic surfactant 3 | | | | | 0.5 | | 0.8 | 0.5 | | | 0.5 | 0.8 | 1.0 |
| Ether type cationic surfactant 4 | | | | | | 0.8 | | | | 0.8 | | | |
| Tertiary amine salt 1 | | | 0.5 | | 0.5 | | | 1.0 | 0.8 | | | | |
| Tertiary amine salt 2 | | | | 1.0 | | | 0.7 | | | | | | |
| Tertiary amine salt 3 | | | | | | 0.8 | | | | 1.0 | | | |
| Tertiary amine salt 4 | | | | | | | | | | | 0.5 | | |
| Tertiary amine salt 5 | | | | | | | | | | | | 1.0 | 0.5 |
| Cetyl trimethyl ammonium chloride | | | | | 0.5 | | | | | | | | |
| Cetanol * | | | 2.0 | 4.0 | 3.0 | 3.5 | 3.5 | 3.0 | 3.5 | 4.0 | 3.5 | 1.5 | |
| Behenyl alcohol | | | 1.0 | | | | | | 1.0 | | | 1.0 | 3.5 |
| Methylpolysiloxane (500 cs) | | | | | | | 1.0 | | 2.0 | | | 1.5 | |
| Amino-modified silicone ** | | | | | | | | | | 1.5 | 1.0 | | |
| pH regulator | | | q.s. | q.s. | q.s. | q.s. | q.s. | q.s. | q.s. | q.s. | q.s. | q.s. | q.s. |
| Purified water | | | Balance | Balance | Balance | Balance | Balance | Balance | Balance | Balance | Balance | Balance | Balance |
| Evaluation results | Upon wetting | Flexibility | B | A | A | A | B | A | A | A | A | A | A |
| | | Smoothness | A | A | A | B | A | B | A | B | A | A | B |
| | After drying | Flexibility | B | B | B | A | B | A | A | A | B | A | A |
| | | Smoothness | A | A | B | A | A | B | A | A | A | A | A |

| | | | | | | | | | | | | | |
|---|---|---|---|---|---|---|---|---|---|---|---|---|---|
| *: a 7:3 (weight ratio) mixture of cetyl alcohol/stearyl alcohol (which will equally apply hereinafter) **: "SM8704C" (product of Toray Dow Corning Silicone) ***: The invention products 7, 8 and 10 (diluted 20 fold with water, 25°C) had pH of 3.5, 4.0 and 4.5, respectively. | | | | | | | | | | | | | |

**Table 4**

| Composition of hair rinse (%) | | | Comparative Examples | | |
|---|---|---|---|---|---|
| | | | 1 | 2 | 3 |
| Ether type cationic surfactant 1 | | | 1.0 | | |
| Ether type cationic surfactant 2 | | | | | |
| Ether type cationic surfactant 3 | | | | | |
| Ether type cationic surfactant 4 | | | | | |
| Tertiary amine salt 1 | | | | | 0.5 |
| Tertiary amine salt 2 | | | | 0.7 | |
| Tertiary amine salt 3 | | | | | |
| Tertiary amine salt 4 | | | | | |
| Tertiary amine salt 5 | | | | | |
| Cetyl trimethylammonium chloride | | | | | 0.5 |
| Cetanol * | | | 2.0 | 3.5 | 3.0 |
| Behenyl alcohol | | | 1.0 | | |
| Methylpolysiloxane (500 cs) | | | | 1.0 | |
| Amino-modified silicone ** | | | | | |
| pH regulator | | | q.s. | q.s. | q.s. |
| Purified water | | | Balance | Balance | Balance |
| | | | | | |
| Evaluation Results | Upon wetting | Flexibility | C | B | B |
| | | Smoothness | C | C | D |
| | After drying | Flexibility | D | C | C |
| | | Smoothness | B | C | D |

| | | | | | |
|---|---|---|---|---|---|
| *: a 7:3 (weight ratio) mixture of cetyl alcohol/stearyl alcohol (which will equally apply hereinafter) **: "SM8704C" (product of Toray Dow Corning Silicone) | | | | | |

### Example 2

A hair rinse having the below-described composition was prepared.

| | (wit.%) |
|---|---|
| Ether type cationic surfactant 1 | 1.0 |
| Tertiary amine salt 2 | 0.5 |
| Cetanol | 3.0 |
| Isopropyl palmitate | 1.0 |
| Dimethylpolysiloxane (500cs) * | 1.0 |
| Propylene glycol | 3.5 |
| Hydroxyethylcellulose | 0.3 |
| Modified alcohol | 2.0 |
| Perfume, methylparaben | q.s. |
| Purified water | Balance |
| Total | 100.0 |

| | |
|---|---|
| *: "SH200C", product of Toray Dow Corning Silicone | |

It has been found that the rinse thus prepared gave good flexibility and smoothness to the hair during the period from wetting to drying and even after drying, and after drying, it gave moist touch, excellent flexibility and smoothness to the hair.

### Example 3

A hair rinse having the following composition was prepared.

| | (wt.%) |
|---|---|
| Ether type cationic surfactant 3 | 0.5 |
| Tertiary amine salt 3 | 1.0 |
| Cetanol | 3.0 |
| Isopropyl palmitate | 2.0 |
| Propylene glycol | 2.0 |
| Hydroxyethylcellulose | 0.2 |
| A 50 wt.% aqueous citric acid solution | 0.2 |
| Perfume, methylparaben | q.s. |
| Purified water | Balance |
| Total | 100.0 |

It has been found that the rinse thus obtained gave good flexibility and smoothness to the hair during the period from wetting to drying and even after drying, and particularly after drying, it gave excellent flexibility and smoothness to the hair.

### Example 4

A leave-on type hair treatment having the below-described composition was prepared.

| | (wt.%) |
|---|---|
| Ether type cationic surfactant 4 | 0.8 |
| Tertiary amine salt 1 | 1.0 |
| Cationic cellulose * | 0.2 |
| Cetanol | 4.0 |
| Diethylene glycol ether | 5.0 |
| Isopropyl myristate | 1.0 |
| Perfume, methylparaben | q.s. |
| Purified water | Balance |
| Total | 100.0 |

| | |
|---|---|
| *: "JR400", product of UCC | |

It has been found that the hair treatment thus obtained gave good flexibility and smoothness to the hair during the period from wetting to drying and even after drying, and particularly after drying, it gave excellent flexibility and smoothness to the hair without giving greasiness thereto.

### Example 5

A hair shampoo having the below-described composition was prepared.

| | (wt.%) |
|---|---|
| Ether type cationic surfactant 1 | 0.3 |
| Tertiary amine salt 1 | 0.2 |
| Sodium polyoxyethylene (3) lauryl ether sulfate | 15.0 |
| Lauryl diethanolamide | 2.0 |
| Cationic polymer ("Merquat 550", product of Calgon) | 0.5 |
| Hydroxysulfobetaine | 3.5 |
| Modified alcohol | 2.0 |
| Perfume, methylparaben | q.s. |
| Purified water | Balance |
| Total | 100.0 |

It has been found that the hair shampoo thus obtained had good foam quality and gave excellent flexibility and smoothness to the hair after drying.

### Example 6

A hair rinse having the below-desccribed composition was prepared

| | (wt.%) |
|---|---|
| Ether type cationic surfactant 3 | 0.8 |
| Tertiary amine salt 4 | 1.0 |
| Stearyl alcohol | 5.5 |
| Propylene glycol | 1.0 |
| Isopropyl myristate | 1.0 |
| Silicone emulsion * | 2.0 |
| Perfume, methylparaben | q.s. |
| Purified water | Balance |
| Total | 100.0 |

| | |
|---|---|
| * : "KM904", product of Shin-etsu Chemical | |

It has been found that the rinse thus obtained had gave good flexibility and smoothness to the hair during from wetting to drying and even after drying, and particularly after drying, it gave moist touch, excellent flexibility and smoothness to the hair.

### INDUSTRIAL APPLICABILITY

The hair cosmetic compositions of the present invention can give good flexibility and smoothness to the hair during the period from wetting to drying and even after drying, and especially after drying, give excellent smoothness to the hair.

## Claims

1. A hair cosmetic composition comprising the following components (A) and (B):
(A) an ether type cationic surfactant represented by the following formula (1): 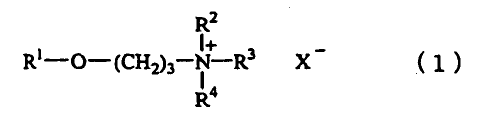
(wherein, R¹ represents a linear or branched alkyl or alkenyl group having 6 to 24 carbon atoms,
R² and R⁴ each represents an alkyl group having 1 to 6 carbon atoms or -(A¹O)ₙH (in which, A¹ represents an alkylene group having 2 to 4 carbon atoms, n stands for 1 to 6, and n pieces of A¹ may be the same or different and arranged in any order),
R³ represents an alkyl group having 1 to 6 carbon atoms, a benzyl group or -(A²O)ₘH (in which A² represents an alkylene group having 2 to 4 carbon atoms, m stands for 1 to 6, and m pieces of A² may be the same or different and arranged in any order), and X⁻ represents an anion), and
(B) at least one compound selected from tertiary amines each represented by the following formula (2): 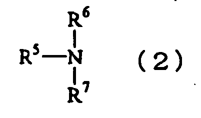
(wherein, R⁵ represents a linear or branched alkyl or alkenyl group having 6 to 24 carbon atoms, or R⁸-Q¹-R⁹ (in which, R⁸ represents a linear or branched alkyl or alkenyl group having 6 to 24 carbon atoms, Q¹ represents a functional group selected from -OCO-, -COO-, -CONH-, -NHCO-, -NH-CO-NH-, and -(A³O)ₛ- (in which A³ represents an alkylene group having 2 to 4 carbon atoms, s stands for 1 to 24, s pieces of A³ may be the same or different and arranged in any order) and R⁹ represents an alkylene group having 1 to 6 carbon atoms),
R⁶ represents a linear or branched alkyl or alkenyl group having 1 to 24 carbon atoms, -(A⁵O)ₖH (in which A⁵ represents an alkylene group having 2 to 4 carbon atoms, k stands for 1 to 6, k pieces of A⁵ may be the same or different and arranged in any order) or R²⁸-Q²-R²⁹ (in which R²⁸ represents a linear or branched alkyl or alkenyl group having 6 to 24 carbon atoms, Q² represents a functional group selected from -OCO-, -COO-, -CONH-, -NHCO-, -NH-CO-NH-, and -(A⁴O)ₜ- (in which A⁴ represents an alkylene group having 2 to 4 carbon atoms, t stands for 1 to 24, t pieces of A⁴ may be the same or different and arranged in any order) and R²⁹ represents an alkylene group having 1 to 6 carbon atoms), and
R⁷ represents a linear or branched alkyl group having 1 to 6 carbon atoms or -(A⁶O)ᵤH (in which A⁶ represents an alkylene group having 2 to 4 carbon atoms, u stands for 1 to 6, u pieces of A⁶ may be the same or different and arranged in any order), and/or salts of the tertiary amines.

2. The hair cosmetic composition of Claim 1, further comprising at least one acid selected from inorganic acids and organic acids.

3. The hair cosmetic composition of Claim 1 or 2, wherein Components (A) and (B) are contained in a total amount of from 0.1 to 20 wt.%.

4. The hair cosmetic composition of any one of Claims 1 to 3, further comprising a surfactant other than Component (A) or (B).

5. The hair cosmetic composition of Claim 4, wherein the surfactant other than Component (A) or (B) is at least one surfactant selected from cationic, anionic, nonionic and amphoteric surfactants.

6. The hair cosmetic composition of Claim 5, wherein the surfactant other than Component (A) or (B) is a cationic surfactant.

## Patentansprüche

1. Haarkosmetikzusammensetzung, umfassend die folgenden Komponenten (A) und (B):
(A) ein kationisches Tensid vom Ethertyp, dargestellt durch die folgende Formel (1): 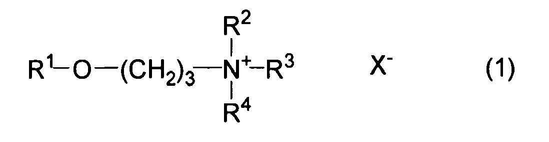
worin R¹ eine lineare oder verzweigte Alkyl- oder Alkenylgruppe mit 6 bis 24 Kohlenstoffatomen ist,
R² und R⁴ jeweils eine Alkylgruppe mit 1 bis 6 Kohlenstoffatomen oder -(A¹O)ₙH sind (worin A¹ eine Alkylengruppe mit 2 bis 4 Kohlenstoffatomen ist, n für 1 bis 6 steht und n Gruppen A¹ gleich oder verschieden und in irgendeiner Reihenfolge angeordnet sein können),
R³ eine Alkylgruppe mit 1 bis 6 Kohlenstoffatomen, Benzylgruppe oder -(A²O)ₘH ist (worin A² eine Alkylengruppe mit 2 bis 4 Kohlenstoffatomen ist, m für 1 bis 6 steht und m Gruppen A² gleich oder verschieden und in irgendeiner Reihenfolge angeordnet sein können), und X⁻ ein Anion ist, und
(B) zumindest eine Verbindung ausgewählt aus tertiären Aminen, jeweils dargestellt durch die folgende Formel (2) 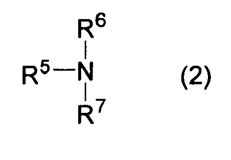
worin R⁵ eine lineare oder verzweigte Alkyl- oder Alkenylgruppe mit 6 bis 24 Kohlenstoffatomen oder -R⁸-Q¹-R⁹ ist (worin R⁸ eine lineare oder verzweigte Alkyl- oder Alkenylgruppe mit 6 bis 24 Kohlenstoffatomen ist, Q¹ eine funktionelle Gruppe ist, ausgewählt aus -OCO-, -COO-, -CONH-, -NHCO-, -NH-CO-NH- und -(A³O)ₛ-(worin A³ eine Alkylengruppe mit 2 bis 4 Kohlenstoffatomen ist, s für 1 bis 24 steht, s Gruppen A³ gleich oder verschieden und in irgendeiner Reihenfolge angeordnet sein können) und R⁹ eine Alkylengruppe mit 1 bis 6 Kohlenstoffatomen ist,
R⁶ eine lineare oder verzweigte Alkyl- oder Alkenylgruppe mit 1 bis 24 Kohlenstoffatomen, -(A⁵O)ₖH (worin A⁵ eine Alkylengruppe mit 2 bis 4 Kohlenstoffatomen ist, k für 1 bis 6 steht, k Gruppen A⁵ gleich oder verschieden und in irgendeiner Reihenfolge angeordnet sein können) oder R²⁸-Q²-R²⁹ ist (worin R²⁸ eine lineare oder verzweigte Alkyl- oder Alkenylgruppe mit 6 bis 24 Kohlenstoffatomen ist, Q² eine funktionelle Gruppe ist, ausgewählt aus -OCO-, -COO-, -CONH-, -NHCO-, -NH-CO-NH- und -(A⁴O)ₜ- (worin A⁴ eine Alkylengruppe mit 2 bis 4 Kohlenstoffatomen ist, t für 1 bis 24 steht, t Gruppen A⁴ gleich oder verschieden und in irgendeiner Reihenfolge angeordnet sein können) und R²⁹ eine Alkylengruppe mit 1 bis 6 Kohlenstoffatomen ist) und
R⁷ eine lineare oder verzweigte Alkylgruppe mit 1 bis 6 Kohlenstoffatomen oder -(A⁶O)ᵤH ist (worin A⁶ eine Alkylengruppe mit 2 bis 4 Kohlenstoffatomen ist, u für 1 bis 6 steht, u Gruppen A⁶ gleich oder verschieden und in irgendeiner Reihenfolge angeordnet sein können) und/oder Salze der tertiären Amine.

2. Haarkosmetikzusammensetzung nach Anspruch 1, weiterhin umfassend zumindest eine Säure, ausgewählt aus anorganischen Säuren und organischen Säuren.

3. Haarkosmetikzusammensetzung nach Anspruch 1 oder 2, worin die Komponenten (A) und (B) in einer Gesamtmenge von 0,1 bis 20 Gew.% enthalten sind.

4. Haarkosmetikzusammensetzung nach einem der Ansprüche 1 bis 3, weiterhin umfassend ein anderes Tensid als die Komponente (A) oder (B).

5. Haarkosmetikzusammensetzung nach Anspruch 4, worin das andere Tensid als die Komponente (A) oder (B) zumindest ein Tensid ist, ausgewählt aus kationischen, anionischen, nichtionischen und amphoteren Tensiden.

6. Haarkosmetikzusammensetzung nach Anspruch 5, worin das andere Tensid als die Komponente (A) oder (B) ein kationisches Tensid ist.

## Revendications

1. Composition cosmétique capillaire comprenant les composants (A) et (B) suivants :
(A) un agent de surface cationique du type éther représenté par la formule (1) suivante: 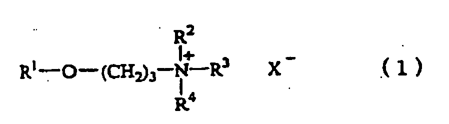
(où, R¹ représente un groupe alkyle ou alcényle linéaire ou ramifié ayant 6 à 24 atomes de carbone,
R² et R⁴ représentent chacun un groupe alkyle ayant 1 à 6 atomes de carbone ou - (A¹O)ₙH (où, A¹ représente un groupe alkylène ayant 2 à 4 atomes de carbone, n représente 1 à 6, et n parties de A¹ peuvent être identiques ou différentes et agencées dans un ordre quelconque),
R³ représente un groupe alkyle ayant 1 à 6 atomes de carbone, un groupe benzyle ou - (A²O)ₘH (où, A² représente un groupe alkylène ayant 2 à 4 atomes de carbone, m représente 1 à 6, et m parties de A² peuvent être identiques ou différentes et agencées dans un ordre quelconque), et X⁻ représente un anion), et
(B) au moins un composé sélectionné à partir d'amines tertiaires représentées chacune par la formule (2) suivante : 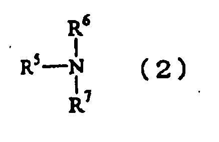
(où, R⁵ représente un groupe alkyle ou alcényle linéaire ou ramifié ayant 6 à 24 atomes de carbone, ou R⁸-Q¹-R⁹ (où, R⁸ représente un groupe alkyle ou alcényle linéaire ou ramifié ayant 6 à 24 atomes de carbone, Q¹ représente un groupe fonctionnel sélectionné à partir de -OCO-, -COO-, -CONH-, -NHCO-, -NH-CO-NH-, et -(A³O)s⁻ (où A³ représente un groupe alkylène ayant 2 à 4 atomes de carbone, s représente 1 à 24, s parties de A³ peuvent être identiques ou différentes et agencées dans un ordre quelconque) et R⁹ représente un groupe alkylène ayant 1 à 6 atomes de carbone),
R⁶ représente un groupe alkyle ou alcényle linéaire ou ramifié ayant 1 à 24 atomes de carbone, -(A⁵O)ₖH (où A⁵ représente un groupe alkylène ayant 2 à 4 atomes de carbone, k représente 1 à 6, k parties de A⁵ peuvent être identiques ou différentes et agencées dans un ordre quelconque) ou R²⁸-Q²-R²⁹ (où, R²⁸ représente groupe alkyle ou alcényle linéaire ou ramifié ayant 6 à 24 atomes de carbone, Q² représente un groupe fonctionnel sélectionné à partir de -OCO-, -COO-, -CONH-, -NHCO-, -NH-CO-NH-, et -(A⁴O)ₜ- (où A⁴ représente un groupe alkylène ayant 2 à 4 atomes de carbone, t représente 1 à 24, t parties de A⁴ peuvent être identiques ou différentes et agencées dans un ordre quelconque) et R²⁹ représente un groupe alkylène ayant 1 à 6 atomes de carbone), et
R⁷ représente un groupe alkyle linéaire ou ramifié ayant 1 à 6 atomes de carbone ou -(A⁶O)ᵤH (où A⁶ représente un groupe alkylène ayant 2 à 4 atomes de carbone, u représente 1 à 6, u parties de A⁶ peuvent être identiques ou différentes et agencées dans un ordre quelconque), et/ou des sels des amines tertiaires.

2. Composition cosmétique capillaire de la revendication 1, comprenant en plus au moins un acide sélectionné à partir d'acides inorganiques ou d'acides organiques.

3. Composition cosmétique capillaire de la revendication 1 ou 2, dans laquelle des composants (A) et (B) sont contenus dans une quantité totale de 0,1 à 20% en poids.

4. Composition cosmétique capillaire de l'une quelconque des revendications 1 à 3, comprenant en plus un agent de surface autre que le composant (A) ou (B).

5. Composition cosmétique capillaire de la revendication 4, dans laquelle l'agent de surface autre que le composant (A) ou (B) est au moins un agent de surface sélectionné à partir d'agents de surface cationiques, anioniques, non-ioniques et amphotères.

6. Composition cosmétique capillaire de la revendication 5, dans laquelle l'agent de surface autre que le composant (A) ou (B) est un agent de surface cationique.
